Europäisches Patentamt

(19) European Patent Office

Office européen des brevets

(11) Publication number: **0 114 086**
**B1**

(12) **EUROPEAN PATENT SPECIFICATION**

(45) Date of publication of patent specification: **24.08.88**

(21) Application number: **84300003.5**

(22) Date of filing: **03.01.84**

(51) Int. Cl.⁴: **C 12 P 7/62, C 08 G 63/06**

(54) Production of beta-hydroxybutyrate polymers.

(30) Priority: **18.01.83 GB 8301344**

(43) Date of publication of application:
**25.07.84 Bulletin 84/30**

(45) Publication of the grant of the patent:
**24.08.88 Bulletin 88/34**

(84) Designated Contracting States:
**BE CH DE FR GB IT LI NL**

(56) References cited:
**EP-A-0 015 669**
**EP-A-0 046 344**

(73) Proprietor: **IMPERIAL CHEMICAL INDUSTRIES PLC**
**Imperial Chemical House Millbank**
**London SW1P 3JF (GB)**

(72) Inventor: **Richardson, Kenneth Raymond**
**18 Ashkirk Road Normanby**
**Middlesbrough Cleveland (GB)**

(74) Representative: **Gratwick, Christopher et al**
**Imperial Chemical Industries PLC Legal**
**Department: Patents PO Box 6 Bessemer Road**
**Welwyn Garden City Herts, AL7 1HD (GB)**

Courier Press, Leamington Spa, England.

## Description

This invention relates to the production of β-hydroxybutyrate polymers. Poly(β-hydroxybutyrate) is a thermoplastic polyester consisting of the repeat units —CH(CH₃) . CH₂ . CO . O— which is accumulated by many micro-organisms, particularly bacteria, as granules within the micro-organism cells as an energy reserve material.

The polymer is conveniently prepared by aerobically cultivating the micro-organism in an aqueous medium on a suitable substrate, i.e. a source of energy and carbon. In order to promote accumulation of the polymer, at least part of the cultivation is preferably conducted under conditions wherein there is a limitation of a nutrient that is essential for growth, i.e. reproduction, of the micro-organism but which is not required for polymer accumulation. Examples of suitable processes are described in EP—A—15669 and 46344.

Polymers containing both β-hydroxybutyrate units and other hydroxy-carboxylate units, such as β-hydroxyvalerate units, can also be produced microbiologically. Thus, as described in EP—A—52459 and 69497, various copolymers can be produced by cultivating the micro-organism on certain substrates, such as propionic acid which gives rise to β-hydroxyvalerate units in the copolymer.

In the following description, by the term PHB, we mean not only β-hydroxybutyrate homopolymer but also such copolymers in which, β-hydroxybutyrate units form at least 40, preferably at least 80, mole % of the polymer chain.

The carbon and energy sources that may be used depend on the nature of the micro-organism: thus autotrophic micro-organisms can utilise carbon dioxide as the carbon source, obtaining their energy by the oxidation of hydrogen, sulphur compounds, nitrogen compounds, or iron compounds. *Alcaligenes eutrophus* is an example of an autotroph that can utilise carbon dioxide and hydrogen. Heterotrophs require organic substances as the carbon and energy sources. Examples of such organic substrates include aliphatic alcohols such as methanol; organic acids or salts thereof e.g. formate, acetates, oxalates, pyruvates, glyoxylates, glycollates; and carbohydrates such as fructose and glucose. Autotrophs may also be able to grow heterotrophically, e.g. on a carbohydrate substrate. Of course the substrate selected must be one that the micro-organism can use: thus while a certain micro-organism may be able to utilise fructose, it may not be able to utilise methanol or glucose.

The cost of the substrate is a significant factor in the overall cost of the production of PHB. Hence it is desirable to increase the yield of PHB from a given amount of substrate.

While the amount of PHB that can be synthesised by the micro-organisms may, in certain cases, be as much as 60% or more by weight of the total biomass, a significant proportion of the substrate is used to produce cell material other than PHB. This non-PHB cell material (hereinafter termed NPCM) represents, in PHB production, a by-product of relatively little value. While it may, in some cases, be used as an animal feedstuff supplement, it often does not have a sufficiently high protein content to be commercially competitive with conventional feedstuff supplements such as soya, especially when the costs involved in obtaining the necessary toxicity clearances for use as a feedstuff are taken into account.

We have found that at least some of the NPCM may be used to supplement the substrate used for cultivation of the micro-organism.

Accordingly we provide a process for the production of PHB comprising cultivating a micro-organism that is capable of accumulating PHB in an aqueous medium on an assimilable substrate, at least part of the cultivation being conducted under conditions that cause said micro-organism to accumulate PHB within the micro-organism cells, and then separating the PHB from the NPCM characterised in that the carbon of at least part of the assimilable carbon source comprises carbon from NPCM separated from the PHB of PHB-containing cells of said micro-organism.

By using NPCM as a source of substrate carbon the amount of fresh substrate required to produce a given amount of PHB is reduced: hence the raw material costs are effectively reduced. Furthermore since the NPCM may also contain various other nutrient materials, e.g. assimilable sources of elements such as nitrogen, phosphorus, sulphur, magnesium and other metals, the amounts of such nutrients that need to be supplied to the micro-organism along with the substrate may also be reduced.

Unless the cultivation is conducted on a considerably smaller scale than that used to produce the NPCM that is re-used, it will be necessary to supplement the re-used NPCM with fresh assimilable substrate. Supplementation of the substrate will also be necessary where a particular substrate, e.g. certain organic acids or derivatives thereof, are required to produce copolymers. Preferably the cultivations are performed on the same scale e.g. by recycling to the cultivation vessel NPCM recovered by separation of the PHB therefrom. In this case the amount of NPCM that is reused is preferably such that the carbon content of the NPCM re-used forms between 5 and 50% by weight of the total carbon required in the assimilable carbon substrate.

The maximum proportion of NPCM that can be re-cycled will depend on, inter alia, the proportion of PHB accumulated by the micro-organism and the efficiency of the conversion of the carbon in the substrate (i.e. re-used NPCM plus fresh substrate) to PHB and to NPCM.

For example 100 g of substrate carbon gives, at 50% carbon conversion of substrate carbon to both PHB carbon and NPCM carbon, 50 g of biomass carbon, of which, at a cell PHB content of 70% by weight, about 37 g will be PHB carbon and about 13 g NPCM carbon. If 70% by weight of this 13 g of NPCM carbon,

2

i.e. about 9 g, is re-used, the amount of re-used carbon is about 9% of the total substrate carbon required. Thus the amount of fresh substrate carbon required is about 91 g. Since this 91 g of fresh substrate carbon, together with the 9 g of re-used NPCM carbon, gives about 37 g of PHB carbon, the overall carbon conversion of fresh substrate to PHB is about 41%. In contrast if no NPCM were to be re-used, the overall carbon conversion of fresh substrate (100 g carbon) to PHB (37 g carbon) would only be 37%. Expressed another way, using glucose as an example of the substrate, an overall carbon conversion to PHB homopolymer of 41% means that about 3.43 kg of glucose is required to produce 1 kg of PHB homopolymer, while, at a carbon conversion of 37%, about 3.77 kg of glucose is required: hence an increase in carbon conversion from 37 to 41% results in a substrate saving of about 9% by weight.

Likewise, if the PHB content of the cells is about 50% by weight, at 50% carbon conversion of substrate carbon to both PHB carbon and to NPCM carbon, 100 g of substrate carbon gives about 27 g of PHB carbon and about 23 g of NPCM carbon. If all of this NPCM carbon is recovered and re-used, the amount of re-used NPCM carbon is 23% by weight of the total substrate carbon required. This corresponds to an overall carbon conversion of fresh substrate carbon to PHB carbon of about 35%: in this case, with no re-use of the NPCM, the carbon conversion to PHB is about 27%.

If a mixture of the recovered NPCM and fresh substrate is fed to the fermenter, the micro-organism may tend to utilise the fresh substrate in preference to metabolising the recovered NPCM. While it may be possible in some cases to overcome this problem by feeding only the recovered NPCM to the fermenter initially, and introducing the fresh substrate only when the recovered NPCM has been utilised, we prefer to treat the NPCM so that it is solubilised to render it more readily assimilable by the micro-organism before it is re-used. This may be effected by subjecting the NPCM to solubilisation, e.g. by hydrolysis, before re-use.

Hydrolysis of the NPCM may be effected enzymatically, for example as part of the PHB extraction process: thus the PHB may be extracted by digesting the micro-organism cells, with a proteolytic enzyme composition, and optionally a lipid solubilising enzyme composition, and then separating the solubilised NPCM from the PHB. Alternatively, where the PHB is extracted from the micro-organism cells by other routes, e.g. solvent extraction, the residual NPCM may be hydrolysed by heating the NPCM suspended in an aqueous medium, preferably at 50 to 150°C. Indeed such a heating step may be desirable where the NPCM has first been solubilised enzymatically. In such a heat treatment stage the hydrolysis is preferably effected at a pH removed from the isoelectric point of the NPCM in order to obtain an acceptable hydrolysis rate. After hydrolysis the solubilised NPCM should be neutralised, if necessary, before re-use as assimilable carbon source. While acidic hydrolysis conditions have proved to give satisfactory hydrolysis, alkaline hydrolysis may be preferable to reduce the amount of nitrogen in the NPCM volatilised as ammonia. In particular the aqueous medium preferably contains 1 to 10% by weight of an alkali such as sodium hydroxide. The aqueous suspension of the NPCM preferably contains 1 to 10% by weight of the NPCM.

Some of the NPCM may be more resistant to solubilisation than the rest. Thus where the separation of the PHB from the NPCM is effected by solubilisation of NPCM, it may not be economic, or possible, to solubilise all of the NPCM enzymatically. Thus most of the NPCM may be solubilised enzymatically, leaving a residue comprising the PHB and some residual NPCM. Other solubilisation techniques, e.g. digestion with a surfactant, may be adopted to solubilise the residual NPCM. Alternatively, or additionally, the PHB may be separated from the residual NPCM by solvent extraction of the PHB.

It is preferred that surfactant solubilised NPCM is not re-used as part of the substrate since the surfactant is liable to interfere with the fermentation.

Likewise, where the NPCM is separated from the PHB by solvent extraction of the PHB, again it may not be economic to solubilise all the recovered NPCM.

It is preferred that the amount of solubilised NPCM that is re-used corresponds to at least 50, particularly 60—85% by weight of the NPCM present in the micro-organism cells prior to separation of the PHB.

The micro-organism may be batch cultured. Under batch culture conditions, the micro-organism will grow, with little or no PHB accumulation, until one or more of the nutrients required for growth becomes exhausted, and then the micro-organism synthesis PHB. Since the re-used NPCM, e.g. a hydrolysate, will often contain a significant amount of the essential requirements for growth, it is preferred to use the recovered NPCM as part or all of the initial substrate feed to the batch fermenter and to add only fresh substrate during the PHB accumulation stage.

Alternatively the micro-organisms may be cultured continuously.

With some micro-organisms PHB may also be accumulated while growth of the micro-organism is taking place: however the amount of PHB so accumulated is generally small and typically is less than about 10% by weight of the cells produced. It is therefore preferred, in order to avoid the necessity for recycling large amounts of the recovered NPCM for efficient substrate usage, to conduct the continuous fermentation under conditions such that the micro-organism accumulates PHB to an extent of at least 25, preferably at least 50% by weight based on the total cell weight. As indicated hereinbefore, a high PHB content also tends to give a higher overall conversion of fresh substrate carbon to PHB carbon. Also a high PHB content is desirable as this renders the subsequent separation of the PHB from NPCM more facile. The requisite PHB content may be achieved by conducting the fermentation under conditions of limitation of one or more of the essential nutrients required for growth but not for PHB accumulation.

The continuous fermentation may be conducted in two stages: the micro-organism is grown in one fermentation vessel and then aqueous medium containing the micro-organism cells is continuously, or intermittently, transferred from the first vessel to a second fermentation vessel to which more substrate is supplied so that PHB accumulation occurs but little or no growth takes place. In such a two stage process the amount of the limiting nutrient required for growth present in the second stage should be small and preferably is only that, if any, present in the aqueous medium transferred to the second fermentation vessel from the first fermentation vessel.

In some cases it may be desirable to operate such a two stage continuous process such that the amount of limiting nutrient required for growth fed to the first fermentation vessel is such that PHB is accumulated, in the first stage, to an extent of at least 25% by weight, based on the total weight of the cells produced in the first vessel. In other cases it may be preferable to operate the first stage under carbon limitation so that virtually no PHB is accumulated by the micro-organism in the first stage.

In addition to the substrate and oxygen (which is generally supplied by injecting air into the aqueous medium in the fermenter), various nutrient salts are required to enable the micro-organism to grow. Thus sources of the following elements in assimilable form, normally as water soluble salts, are generally required: nitrogen, phosphorus, sulphur, potassium, sodium, magnesium, calcium, and iron, together with traces of elements such as manganese, zinc and copper. While it may be possible to induce PHB accumulation by restricting the supply of oxygen to the fermenter, it is preferred to restrict the amount of one or more of the nutrient salts. The most practical elements to limit are nitrogen, phosphorus, sulphur or, less preferably, magnesium. Of these it is most preferred to restrict the amount of nitrogen (which is conveniently supplied as an ammonium salt) or phosphorus. The amount of assimilable nitrogen required varies from micro-organism to micro-organism but generally is within the range 8—15% by weight of the NPCM produced.

The recovered NPCM used as part of the substrate will generally contain some of the limiting nutrient, e.g. assimilable nitrogen, and so, when operating a continuous two stage fermentation process, the recovered NPCM is preferably used only in the first stage, even then generally only as part of the first stage substrate, and only fresh substrate is fed to the second stage fermentation vessel. Where a continuous two stage process is operated, the carbon of the recovered NPCM preferably represents 40 to 80% by weight of the carbon required in the first stage, particularly where the first stage is conducted under carbon limitation conditions.

The proportion of PHB accumulated in the cells depends, inter alia, on the average residence time of the medium containing the suspended micro-organism in the fermenter. Providing sufficient substrate is supplied, the greater the residence time the greater will be the PHB content. To obtain the optimum carbon conversion at any desired PHB content, the amount of substrate employed should be kept to the minimum that is consistent with economic operation. In general it is desirable to have a slight excess of substrate so that there is a small concentration of residual substrate in the aqueous medium removed from the fermenter.

While the micro-organisms may be capable of accumulating relatively high proportions of PHB, e.g. up to 75—80% by weight based on the total weight of the cells, the residence time to achieve such a high PHB content will generally be uneconomically lengthy in a single stage continuous fermentation process. Hence in such a process it is preferred that the residence time is such that the PHB content is less than 70%, and in particular is between 35 and 65%, by weight.

The process is preferably operated so that the dry weight of the PHB-containing cells is at least 5 g per litre of aqueous medium. Hence if, for example, it is desired to produce 10 g per litre of PHB-containing cells having a PHB content of 40% by weight, the amount of the limiting nutrient must be that required to support the growth of 6 g per litre of NPCM thus, if nitrogen is employed as the growth limiting nutrient, since the nitrogen content of PHB free bacterial cells will generally be within the range 8—15% by weight, the amount of assimilable nitrogen required would be between about 0.5 and 1 g per litre; the precise amount depending on inter alia, the micro-organism employed.

The fermentation may be conducted under the conditions e.g. pH, temperature, and degree of aeration (unless oxygen is utilised as the limiting nutrient) conventionally used for the micro-organism. Likewise the amounts of nutrient salts (other than the growth limiting nutrient whose amount may be determined following the considerations outlined hereinbefore) employed may be those normally used for growth of the micro-organism.

Where a two-stage fermentation process is employed, in the second stage the PHB content of the cells is preferably increased to between 50 and 80% by weight.

The fresh substrate employed in the second stage may be the same as, or different to, that used in the first stage. In some cases the overall efficiency of the process may be increased by utilising in the first stage as the substrate a mixture of the recovered NPCM, and a material such as a carbohydrate, that is fairly efficiently converted to cell material while in the second stage the substrate is a material, such as an organic acid or salt thereof, e.g. an acetate, which is converted efficiently to PHB but on which the micro-organism grows less efficiently. Where copolymers are required the second stage substrate should include that material that gives rise to the copolymer units.

After fermentation has proceeded to the desired extent, the PHB is extracted from the micro-organism cells. Preferably the aqueous cell suspension is first concentrated, e.g. by centrifugation. The separated aqueous medium may be re-used, e.g. recycled to the fermenter, after such sterilisation as may be necessary, so that any residual substrate in the separated aqueous medium can be re-used and hence the overall carbon conversion improved. The PHB is then extracted from the cells in the concentrated suspension. A variety of methods have been proposed, generally involving contact of the cells, in some cases after one or more pretreatment steps, such as cell disruption, with a solvent in which the PHB is soluble. Examples of solvents that have been proposed include pyridine (see US—A—3036959); methylene chloride/ethanol mixtures (see US—A—3044942); chloroform (see US—A—3275610); cyclic carbonates (see US—A—4101533); and 1,2-dichloroethane (see EP—A—14490 and 15123).

One preferred extraction method comprises the following steps:

(i)   spray drying the concentrated suspension;

(ii)  extraction of lipids by contacting the dried cells, e.g. under reflux conditions, with a solvent such as methanol or acetone in which PHB is insoluble;

(iii) separating, e.g. by filtration, the lipid-free cells from the lipid containing solution;

(iv) extracting the PHB by contacting the lipid-free cells, e.g. under reflux conditions, with a solvent in which the PHB is soluble. 1,2-Dichloroethane and chloroform are particularly suitable solvents;

(v)  separating, e.g. by filtration, the solution of the PHB in the extraction solvent from the cell residue;

(vi) precipitating the PHB from the solution by adding the latter to a liquid, e.g. a methanol/water mixtures in which the PHB is not soluble; and

(vii) separating the precipitated PHB therefrom, e.g. by filtration.

Such a process is described in aforementioned EP—A—15123.

Other separation processes, for example an enzymatic digestion process as described hereinbefore, may of course be employed. Where the NPCM is separated from the PHB after contact with a PHB-extraction solvent, as the latter are often toxic to micro-organisms, it is generally necessary to remove, e.g. by washing or drying, any of the solvent that may be occluded in the NPCM before re-use of the latter. Drying may simply be effected by heating the NPCM in a suitable oven. The residual solvent evolved may be recovered and used for extraction of PHB from a further quantity of cells. Alternatively, where the NPCM is hydrolysed before re-use, the residual solvent may, in some cases, be volatilised during the hydrolysis.

Micro-organisms that may be used to produce PHB include:

Nocardia, e.g. *N. salmonicolor, N. asteroides, N. opaca, N. corallina, N. rubra;*

Azotobacter, e.g. *A. chroococcum, A. beijerinckii, A. agilis, A. indicus;*

Bacillus, e.g. *B. megaterium, B. mycoides, B. subtilis, B. cereus, B. anthracis;*

Micrococcus, e.g. *M. halodenitrificans, M. denitrificans;*

Rhizobium, e.g. *Rh. leguminosarum, Rh. phaseoli, Rh. trifoli, Rh. lupini, Rh. japonicum;*

Rhodospirillum, e.g. *R. rubrum, R. fulrum;*

Methylobacterium, e.g. *Me. organophilum* such as the strains designated by NCIB No's 11482—11488 inclusive and as described in EP—A—15669;

Pseudomonas, e.g. *P. rosea, P. saccharophila, P. AM 1;*

Hydrogenomonas, e.g. *H. ruhlandii, H. eutropha* (otherwise known as *Alcaligenes eutrophus*) such as strain H16 widely employed in academic studies of this species, see e.g. J. General Microbiology (1979) *115* pages 185—192, and which is available as ATCC strain 17699, and mutants of strain H16 such as the glucose utilising mutants designated by NICB No's 11597—11600 inclusive.

NCIB No. refers to the number designated to the strain deposited with the National Collection of Industrial Bacteris Torry Research Station, Aberdeen, Scotland.

ATCC No. refers to the number designated to the strain deposited with the American Type Culture Collection, 12301 Park Lawn Drive, Rockville, Maryland 20852, USA.

The invention is illustrated by the following Example.

Example

*Alcaligenes eutrophus* NCIB 11599 was aerobically cultivated on glucose as a carbon and energy source in an aqueous medium containing a restricted amount of assimilable nitrogen to produce a suspension of cells containing approximately 50% by weight of PHB homopolymer. The PHB was extracted from the cells by spray drying the suspension, refluxing the spray dried cells with chloroform, and filtering the cell residue from the solution of PHB in chloroform. The PHB was precipitated from the filtered solution by adding the latter to a methanol/water mixture.

The cell residue filtered from the solution was suspended in 6 N hydrochloric acid to give a suspension containing about 200 g/l of cell residue. The suspension was then refluxed for 24 hours to hydrolyse the cell residue. The resulting mixture was neutralised to pH 7 with sodium hydroxide, allowed to cool, and then filtered. The hydrolysate was diluted to 20 times its volume and analysed (aqueous medium A).

100 Volumes of the diluted hydrolysate was sterilised by heating for 1 hour at 121°C and then inoculated with 0.5 volume of an *Alcaligenes eutrophus* NCIB 11599 culture of 10 g/l concentration. The mixture was cultivated aerobically at 34°C for 48 hours: the mixture then contained approximately 3 g/l of micro-organism cells.

The cells were separated from the aqueous medium by centrifuging: the cells and residual aqueous medium (aqueous medium B) were analysed.

The analyses are shown in the following Table.

TABLE

| | Aqueous medium | | Cells |
|---|---|---|---|
| | A (mg/100 ml) | B (mg/100 ml) | (% by weight) |
| Total organic carbon | 430 | 68 | 46.3 |
| Carbohydrate | 0.05 | 0.05 | NM |
| PHB | — | — | approx. 40 |
| $NH_4^+$ | 5.8 | 0.006 | NM |
| Aspartic acid | 29 | 3 | 4.0 |
| Threonine | 15 | 2 | 2.2 |
| Serine | 10 | 3 | 1.7 |
| Glutamic acid | 37 | 7 | 5.4 |
| Proline | 12 | — | 2.0 |
| Glycine | 17 | 3 | 2.4 |
| Alanine | 33 | 3 | 4.1 |
| Valine | 20 | 3 | 2.8 |
| Methionine | 6 | 1 | 1.1 |
| Isoleucine | 13 | 2 | 1.9 |
| Leucine | 25 | 2 | 3.6 |
| Tyrosine | 10 | 2 | 1.7 |
| Phenylalanine | 14 | 2 | 2.0 |
| Histidine | 6 | 1 | 1.0 |
| Lysine | 15 | 3 | 2.9 |
| Argenine | 20 | 17 | 3.9 |
| Total amino acids | 282 | 54 | 36.4 |
| Nucleic acids | 110 | 49 | NM |
| Total nitrogen | NM | NM | 7.0 |

NM=not measured.

It is seen that about 84% by weight of the carbon in the carbon in the hydrolysate was utilised by the micro-organism.

**Claims**

1. A process for the production of a β-hydroxybutyrate polymer having at least 40 mole % of β-hydroxybutyrate units in the polymer chain comprising cultivating a micro-organism that is capable of accumulating said polymer in an aqueous medium on an assimilable carbon source, at least part of the cultivation being conducted under conditions that cause said micro-organism to accumulate said polymer within the micro-organism cells, and then separating said polymer accumulated in said micro-organism cells from the other cell material, characterised in that the carbon of at least part of said assimilable carbon source comprises carbon from cell material separated from the β-hydroxybutyrate polymer of β-hydroxybutyrate polymer-containing cells of said micro-organism.

2. A process according to claim 1 characterised in that said at least part of the assimilable carbon source comprises solubilised cell material separated from the β-hydroxybutyrate polymer of β-hydroxybutyrate polymer-containing cells of said micro-organism.

3. A process according to claim 2 characterised by solubilising at least part of the cell material, other than said β-hydroxybutyrate polymer, of said micro-organism cells produced in the cultivation step, and recycling said solubilised cell material to the cultivation step as part of the assimilable carbon source used therein.

4. A process according to claim 2 or claim 3 characterised in that said solubilisation comprises treatment of the cell material with an enzyme composition.

5. A process according to claim 4 characterised in that said solubilisation comprises digesting said β-hydroxybutyrate polymer-containing cells with a proteolytic enzyme composition and thereafter separating the solubilised cell material from the β-hydroxybutyrate polymer-containing residue.

6. A process according to any one of claims 2 to 5 wherein said solubilisation comprises heating said cell material in an aqueous medium at 50 to 150°C.

7. A process according to any one of claims 1 to 6 wherein the cultivation of the micro-organism is conducted continuously.

6

**0 114 086**

8. A process according to claim 7 wherein the micro-organism is first cultivated in an aqueous medium on an assimilable carbon source in a first vessel and then the aqueous medium containing the micro-organism cells is continuously or intermittently transferred to a second vessel wherein cultivation of said micro-organism on an assimilable carbon source is continued in the presence of such a restricted amount of a nutrient required for growth, but not polymer accumulation, that said polymer is accumulated by said micro-organism during the cultivation in said second vessel, characterised in that only the carbon of the assimilable carbon source employed for the cultivation in said first vessel comprises the carbon derived from the cell material separated from the β-hydroxybutyrate polymer of β-hydroxybutyrate polymer-containing cells of said micro-organism.

**Patentansprüche**

1. Verfahren zur Herstellung eines β-Hydroxybutyratpolymers mit mindestens 40 Mol.-% an β-Hydroxybutyrat-Einheiten in der Polymerkette, das folgendes umfaßt: Kultivieren eines Mikroorganismus, der fähig ist, das Polymer in einem wässrigen Medium auf einer assimilierbaren Kohlenstoffquelle anzusammeln, wobei mindestens ein Teil der Kultivierung unter Bedingungen durchgeführt wird, die diesen Mikroorganismus veranlassen, das Polymer innerhalb der Zellen des Mikroorganismus anzusammeln, und danach Trennen des in den Zellen des Mikroorganismus angesammelten Polymers vom übrigen Zellmaterial, dadurch gekennzeichnet, daß der Kohlenstoff mindestens eines Teiles der assimilierbaren Kohlenstoffquelle Kohlenstoff aus Zellmaterial umfaßt, das von dem β-Hydroxybutyratpolymer der β-Hydroxybutyratpolymer enthaltenden Zellen des Mikroorganismus abgetrennt wurde.

2. Verfahren nach Anspruch 1, dadurch gekennzeichnet, daß mindestens ein Teil der assimilierbaren Kohlenstoffquelle aufgeschlossenes Zellmaterial umfaßt, das von dem β-Hydroxybutyratpolymer der β-Hydroxybutyratpolymer enthaltenen Zellen des Mikroorganismus abgetrennt wurde.

3. Verfahren nach Anspruch 2, dadurch gekennzeichnet, daß mindestens ein Teil des von dem β-Hydroxybutyratpolymer verschiedenen Zellmaterials der im Kultivierungsschritt hergestellten Mikroorganismuszellen aufgeschlossen wird, und daß das aufgeschlossene Zellmaterial in den Kultivierungsschritt als Teil der darin verwendeten assimilierbaren Kohlenstoffquelle zurückgeführt wird.

4. Verfahren nach Anspruch 2 oder 3, dadurch gekennzeichnet, daß dieser Aufschluß die Behandlung des Zellmaterials mit einer Enzymzusammensetzung umfaßt.

5. Verfahren nach Anspruch 4, dadurch gekennzeichnet, daß der Aufschluß die Umsetzung der β-Hydroxybutyratpolymer enthaltenen Zellen mit einer proteolytischen Enzymzusammensetzung und danach die Trennung des aufgeschlossenen Zellmaterials von dem β-Hydroxybutyratpolymer enthaltenden Rest umfaßt.

6. Verfahren nach einem der Ansprüche 2 bis 5, in dem der Aufschluß das Erhitzen des Zellmaterials in einem wässrigen Medium bei 50 bis 150°C umfaßt.

7. Verfahren nach einem der Ansprüche 1 bis 6, in dem die Kultivierung des Mikroorganismus kontinuierlich durchgeführt wird.

8. Verfahren nach Anspruch 7, in dem der Mikroorganismus zuerst in einem wässrigen Medium auf einer assimilierbaren Kohlenstoffquelle in einem ersten Behälter kultiviert wird und danach das die Mikroorganismuszellen enthaltende wässrige Medium kontinuierlich oder diskontinuierlich in einen zweiten Behälter übergeführt wird, in dem die Kultivierung des Mikroorganismus auf einer assimilierbaren Kohlenstoffquelle in Gegenwart einer solch begrenzten für das Wachstum aber nicht für Polymeranhäufung erforderlichen Menge eines Nährstoffes weitergeführt wird, so daß das Polymer durch den Mikroorganismus während der Kultivierung in den zweiten Behälter angesammelt wird, dadurch gekennzeichnet, daß nur der bei der Kultivierung in dem ersten Behälter verwendete Kohlenstoff der assimilierbaren Kohlenstoffquelle den Kohlenstoff umfaßt, der sich von dem Zellmaterial ableitet, das von dem β-Hydroxybutyratpolymer der β-Hydroxybutyratpolymer enthaltenden Zellen des Mikroorganismus abgetrennt wurde.

**Revendications**

1. Procédé pour la production d'un polymère d'acide β-hydroxybutyrique comprenant au moins 40 moles % d'unités β-hydroxybutyrate dans la chaîne du polymère, qui comprend la culture d'un micro-organisme qui est capable d'accumuler ce polymère dans un milieu aqueux sur une source de carbone assimilable, au moins une partie de la culture étant conduite dans des conditions qui font que le micro-organisme accumule le polymère dans les cellules du micro-organisme, puis la séparation entre le polymère accumulé dans les cellules du micro-organisme et la matière cellulaire autre, caractérisé en ce que le carbone d'au moins une partie de la source de carbone assimilable comprend du carbone de la matière cellulaire séparée du polymère d'acide β-hydroxybutyrique des cellules de ce microorganisme contenant le polymère d'acide β-hydroxybutyrique.

2. Procédé suivant la revendication 1, caractérisé en ce que cette partie de la source de carbone assimilable comprend de la matière cellulaire solubilisée séparée du polymère d'acide β-hydroxybutyrique des cellules de ce micro-organisme contenant le polymère d'acide β-hydroxybutyrique.

7

3. Procédé suivant la revendication 2, caractérisé par la solubilisation d'au moins une partie de la matière cellulaire, autre que le polymère d'acide β-hydroxybutyrique, des cellules de ce micro-organisme produites au cours du stade de culture, et le recyclage de la matière cellulaire solubilisée au stade de culture comme partie de la source de carbone assimilable qui y est utilisée.

4. Procédé suivant la revendication 2 ou 3, caractérisé en ce que la solubilisation comprend le traitement de la matière cellulaire à l'aide d'une composition enzymatique.

5. Procédé suivant la revendication 4, caractérisé en ce que la solubilisation comprend la digestion des cellules contenant le polymère d'acide β-hydroxybutyrique à l'aide d'une composition enzymatique protéolytique et ensuite la séparation entre la matière cellulaire solubilisée et le résidu contenant le polymère d'acide β-hydroxybutyrique.

6. Procédé suivant l'une quelconque des revendications 2 à 5, dans lequel la solubilisation comprend le chauffage de la matière cellulaire dans un milieu aqueux de 50 à 150°C.

7. Procédé suivant l'une quelconque des revendications 1 à 6, dans lequel la culture du micro-organisme est exécutée en marche continue.

8. Procédé suivant la revendication 7, dans lequel le micro-organisme est cultivé d'abord dans un milieu aqueux sur une source de carbone assimilable dans un premier récipient et le milieu aqueux contenant les cellules du micro-organisme est ensuite transféré de façon continue ou intermittente dans un second récipient dans lequel la culture du micro-organisme sur une source de carbone assimilable est poursuivie en présence d'une quantité d'un nutriment nécessaire pour la croissance, mais non pour l'accumulation du polymère, qui est restreinte de façon que le polymèr soit accumulé par le micro-organisme pendant la culture dans le second récipient, caractérisé en ce que seul le carbone de la source de carbone assimilable utilisée pour la culture dans le premier récipient comprend le carbone provenant de la matière cellulaire séparée du polymère d'acide β-hydroxybutyrique des cellules de ce micro-organisme contenant le polymère d'acide β-hydroxybutyrique.